# EUROPEAN PATENT APPLICATION

(11) **EP 3 995 071 A1**
(43) Date of publication of application: **11.05.2022**
(21) Application number: 20205912.7
(22) Date of filing: 05.11.2020
(51) Int. Cl.: A61B 5/00, A61B 5/16

(54) **ENHANCING SLEEP THERAPY COMPLIANCE**

(71) Applicant: Koninklijke Philips N.V., 5656 AG Eindhoven (NL)
(72) Inventor: WEIJSEN, Tim, 5656 AE Eindhoven (NL); LEUFKENS, Timmy Robertus Maria, 5656 AE Eindhoven (NL); WESTERINK, Joanne Henriëtte Desirée Monique, 5656 AE Eindhoven (NL); MENA BENITO, Maria Estrella, 5656 AE Eindhoven (NL); PFUNDTNER, Stefan, 5656 AE Eindhoven (NL); VAN EE, Raymond, 5656 AE Eindhoven (NL)
(74) Representative: Philips Intellectual Property & Standards

(57) **Abstract**

A computer-implemented method (100) of producing a sleep evaluation for a user for use with a sleep therapy is disclosed. The method comprises, with a processor arrangement (12), receiving (122) a perceived value of a sleep parameter from the user, said sleep parameter corresponding to sleep behavior of said user at a point in time during said sleep therapy; receiving (124) sleep monitoring data from an electronic sleep monitor, said sleep monitoring data incorporating an objective value of said sleep parameter at said point in time; and, in case of the perceived value being different to the objective value, processing the sleep monitoring data such as to augment (132) said objective value into a further value of said sleep parameter to reduce the discrepancy between the perceived value and the objective value; and producing (134) a sleep evaluation output for the user comprising at least the further value of said sleep parameter. Also disclosed is a computer program product for implementing such a method (100) on a processor arrangement (12) and a sleep monitoring system (1) adapted to implement such a method (100).

## Description

### FIELD OF THE INVENTION

The present invention relates to a computer-implemented method of producing a sleep evaluation for a user for use in a sleep therapy., the method comprising, with a processor arrangement, receiving a perceived value of a sleep parameter from the user, said sleep parameter corresponding to sleep behavior of said user at a point in time during said sleep therapy, receiving sleep monitoring data from an electronic sleep monitor, said sleep monitoring data corresponding to sleep behavior of said user at said point in time and extracting an objective value of said sleep parameter from the received sleep monitoring data.

The present invention further relates to a computer program product for implementing such a method on a computer.

The present invention further relates to a sleep monitoring system adapted to implement such a method.

### BACKGROUND OF THE INVENTION

Chronic insomnia is a widespread problem, affecting about 10 percent of the adult population globally. Insomnia does not only involve unsatisfactory sleep, but also leads to daytime complaints such as fatigue, attentional disturbances and mood disturbances. Paradoxical insomnia, which is sometimes referred to as sleep state misperception, is the reporting of severe insomnia without corroborative objective evidence of sleep disturbance or significant impairment of daytime function. Different types of paradoxical insomnia can be distinguished, for example with respect to total sleep time (TST), wake after sleep onset (WASO) or sleep onset latency (SOL). Sufferers from paradoxical insomnia have a marked propensity to underestimate sleep duration and overestimate wakefulness relative to objective (polysomnographic or sleep tracker) measures.

The treatment of chronic insomnia consists of initially diagnosing and treating the underlying medical or psychological problems. Cognitive behavioral therapy for insomnia (CBT-I) is a treatment option with strong support for its effectiveness. Computerized cognitive behavioral therapy (cCBT-I) is an innovative method of delivering services via digital platforms to patients with psychological disorders like insomnia. This method improves sleep in individuals with insomnia and co-occurring mental illness. These methods rely on subjective reports from the insomniac where data is collected in so-called sleep wake diaries, which are used e.g. for setting a bedtime window during sleep restriction therapy or assessing overall therapy effect.

The American Academy of Sleep Medicine's "Practice parameters for using polysomnography (PSG)", Iber, Conrad & Ancoli-Israel, Sonia & Chesson, A.L. & Quan, Stuart. (2007), the AASM Manual for the Scoring of Sleep and Associated Events: Rules, Terminology and Technical Specifications. Westchester, IL: American Academy of Sleep Medicine, recommends against the use of routine PSG in clinical evaluation of transient or chronic insomnia. It states that "insomnia is primarily diagnosed by clinical evaluation, through detailed medical, psychiatric and sleep history". Still, modern digital CBT programs for insomnia (cCBT-i) tend to include objective measures of sleep to tailor the treatment and track progress during the course of the treatment.

Recent evidence suggests that individuals who follow a cCBT-I program with a connected sleep tracker report more severe insomnia complaints, report more use of sleep medication and suffer from decreased work productivity than individuals who manually complete sleep diaries users of devices. Interestingly, the interaction with the cCBT-I program increases when users connect a sleep tracker, yielding an advantage of connected sleep trackers to cCBT-I programs. However, a major issue of these self-tracking devices is that they tend to overestimate sleep duration and underestimate the amount of wake, thus making the gap between user-perceived and device-reported sleep duration even bigger. Moreover, in patients suffering from paradoxical insomnia, the disagreement of objective measures with the subjective experience of symptoms of a patient (increased by the tendency of self-trackers to overestimate sleep duration) may lead to distrust in the sleep data collected by a tracker and the connected program, affecting adherence & treatment outcome in a negative manner. Hence, there is a need to mitigate the potential negative impact of the use of such sleep trackers on treatment outcomes achieved by individuals following cCBT-I programs incorporating such sleep trackers.

### SUMMARY OF THE INVENTION

The present invention seeks to provide a computer-implemented method of producing a sleep evaluation for a user as part of a sleep therapy in which the likelihood of the user's compliance with the sleep therapy is improved.

The present invention further seeks to provide a computer program product for implementing such a method on a computer.

The present invention yet further seeks to provide a sleep monitoring system adapted to implement such a method.

According to an aspect, there is provided a computer-implemented method of producing a sleep evaluation for a user for use with a sleep therapy, the method comprising, with a processor arrangement, receiving a perceived value of a sleep parameter from the user, said sleep parameter corresponding to sleep behavior of said user at a point in time during said sleep therapy; receiving sleep monitoring data from an electronic sleep monitor, said sleep monitoring data incorporating an objective value of said sleep parameter at said point in time; and, in case of the perceived value being different to the objective value, processing the sleep monitoring data such as to augment said objective value into a further value of said sleep parameter to reduce the discrepancy between the perceived value and the objective value; and producing a sleep evaluation output for the user comprising at least the further value of said sleep parameter.

The present invention is based on the insight that compliance with a sleep therapy such as cCBT-I to treat paradoxical insomnia improves when a patient is presented with a value of a sleep parameter from the sleep monitor that more closely resembles the patient's own perception of the value of this sleep parameter, thereby increasing trust of the patient in the sleep therapy and improving the likelihood of the patient's compliance with the tasks and challenges set for the patient as part of the sleep therapy. To this end, the method augments the objective value of the sleep parameter that is obtained from the sleep monitor such that the discrepancy between the objective value produced by the sleep monitor more closely resembles the value of the sleep parameter as perceived by the patient, such that when the augmented objective value, i.e. the further value, is reported back to the patient, e.g. in the form of a legible output with in app running on an electronic device such as a smart phone or the like, the patient is reassured that his or her symptoms are recognized by the system implementing the computerized sleep therapy. Such a legible output for example may form an entry of a sleep diary kept by the patient.

The sleep evaluation output for the user may comprise the perceived value of the sleep parameter and the augmented objective value, i.e. the further value, of the sleep parameter such that the user (patient) is presented with both his or her perceived value of the sleep parameter as well as the value of sleep parameter obtained with the sleep monitor and augmented by the processor arrangement as previously explained, e.g. as part of a sleep diary or the like.

Preferably, said augmenting is a function of the point in time during said sleep therapy such that an amount by which said objective value is augmented into the further value is reduced over time such that as the sleep therapy progresses, the augmented measured value of the sleep parameter more closely resembles its objective value, e.g. a value obtained in accordance with recognized or recommended method for determining the value, thereby giving the user (patient) additional confidence that the sleep therapy is working, as the patient's own perception of his or her quality of sleep typically will more closely reflect the actual quality of sleep as the sleep therapy progresses.

In an embodiment, augmenting the objective value of said sleep parameter into the further value comprises augmenting a threshold value for recognizing a data pattern in the sleep monitoring data from which the objective value of the sleep parameter is determined. Typically, the occurrence of a data pattern, e.g. a data pattern indicating a state of sleep, may be used to determine whether the patient has entered a state of sleep or awakening, in particular where the characteristic data pattern is observed for a contiguous period of time as defined by a threshold value. For instance, augmenting said threshold value may comprise altering recommended threshold value into a further threshold value producing a further value of the sleep parameter that more closely resembles the perceived value of the sleep parameter compared to the objective value of the sleep parameter produced with the recommended threshold value in order to improve the patient's confidence in the data produced by the sleep monitor.

In another embodiment, the method further comprises receiving a series of further perceived values of a sleep parameter from the user, each of said further perceived values of said sleep parameter corresponding to sleep behavior of said user at different points in time during a calibration period preceding said sleep therapy; receiving a series of further sleep monitoring data from the electronic sleep monitor, each instance of said further sleep monitoring data corresponding to sleep behavior of said user at one of said different points in time during said calibration period; extracting a further objective value of said sleep parameter from each instance of the received sleep monitoring data; determining a difference between the further perceived values and the further objective values of the sleep parameter for each of said points in time during said calibration period; and augmenting the objective value of said sleep parameter based on said determined difference.

In this embodiment, a calibration or benchmark period may precede the actual sleep therapy such that the system implementing the method can learn the magnitude of the discrepancy between the user perceived value of the sleep parameter and the objective value of the sleep parameter obtained with the sleep monitor to improve the accuracy of the augmentation of the objective value of the sleep parameter into the further value during the sleep therapy in order to increase patient confidence in the sleep therapy as previously explained. Such a determined difference for example may be one of an average difference, a maximum difference and a median difference.

In yet another embodiment, augmenting the objective value of said sleep parameter into the further value comprises retrieving an optimal further value from a database containing sleep therapy outcomes as a function of different further values used for an actual difference between the perceived value and the objective value of the sleep parameter in said sleep therapies. In this embodiment, knowledge of treatment success with other patients is leveraged to increase the likelihood of a successful sleep therapy for the patient by retrieving applied augmented values of the further value of the sleep parameter from a database that have led to successful treatment of other paradoxical insomnia patients for which a similar discrepancy between the user perceived value and the further value of the sleep parameter has been established.

In a further refinement, the method may further comprise retrieving the optimally augmented further value from a database containing sleep therapy outcomes as a function of different augmented further values used for the actual difference between the perceived value and the objective value of the sleep parameter in different sleep therapies; and patient characteristics matching the type of sleep disorder of the user to further improve the likelihood of augmenting the further value of the sleep parameter in a manner that will lead to compliance of the patient with the sleep therapy.

In yet another embodiment, augmenting the objective value of said sleep parameter into the further value is at least in part based on a known systematic inaccuracy in the sleep monitoring data produced by the sleep monitor. In this embodiment, knowledge about the nature of the sleep monitoring data produced by the sleep monitor, i.e. leading to systematic under- or overestimation of the actual value of the sleep parameter may be leveraged to augment (correct) the objective value using such a quantified systematic error to reduce the discrepancy between the value of the sleep parameter as perceived by the user (patient) and the objective value of the sleep parameter obtained with the sleep monitor.

The sleep parameter is one of sleep onset latency, total sleep time and wake after sleep onset.

In accordance with another aspect, there is provided a computer program product comprising a computer readable storage medium having computer readable program instructions embodied therewith for, when executed on the processor arrangement, cause the processor arrangement to implement the method of any of the herein described embodiments. Such a computer program product may be used to adapt a computer, that is, an electronic device comprising a processor arrangement including one or more processor elements, such that the computer is configured to implement the method in accordance with the herein described embodiments.

In accordance with yet another aspect, there is provided a sleep monitoring system comprising a processor arrangement and the aforementioned computer program product, wherein the processor arrangement is adapted to execute the computer readable program instructions of said computer program product. Such a sleep monitoring system may be used to implement a sleep therapy for patients suffering from paradoxical insomnia, where the augmentation of the objective values of the sleep parameter obtained from a sleep monitor such as to reduce the discrepancy between these values and the user perceived values of the sleep parameters, in particular during the initial stages of the sleep therapy, increases the likelihood of compliance of such patients with the sleep therapy as the impression is created that the system produces (augmented) further values of the monitored sleep parameter that appear in agreement with the patient's own perception, thus improving the patient's trust in the system.

Preferably, the sleep monitoring system further comprises at least one of a user interface adapted to be communicatively coupled to the processor arrangement for providing the processor arrangement with a perceived value of a sleep parameter from a user, said sleep parameter corresponding to sleep behavior of said user at a point in time during a sleep therapy; and a sleep monitor adapted to be communicatively coupled to the processor arrangement for providing the processor arrangement with sleep monitoring data corresponding to sleep behavior of said user at said point in time. The processor arrangement and the user interface may form part of the same electronic device.

### BRIEF DESCRIPTION OF THE DRAWINGS

Embodiments of the invention are described in more detail and by way of nonlimiting examples with reference to the accompanying drawings, wherein:
FIG. 1 schematically depicts a sleep monitoring system according to an embodiment;
FIG. 2 schematically depicts a sleep monitoring system according to another embodiment;
FIG. 3 is a flowchart of a sleep monitoring method according to an embodiment;
FIG. 4 is a flowchart of an aspect of a sleep monitoring method according to an embodiment;
FIG. 5 is a graphical representation of the difference between a perceived value (solid lines) and a measured value (dashed lines) of a sleep parameter for healthy sleepers (bold lines) and a paradoxical insomniac (thin lines).
FIG. 6 is a flowchart of a sleep monitoring method according to another embodiment;
FIG. 7 is a graph depicting a difference in objective and perceived values of a sleep parameter for a paradoxical insomniac over the course of a 6-week sleep therapy program;
FIG. 8 is a graph depicting a difference in objective and perceived values of a sleep parameter for a particular paradoxical insomniac over the course of a 6-week sleep therapy program; and
FIG. 9 is a graph depicting a difference in objective and perceived values of a sleep parameter for another particular paradoxical insomniac over the course of a 6-week sleep therapy program.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

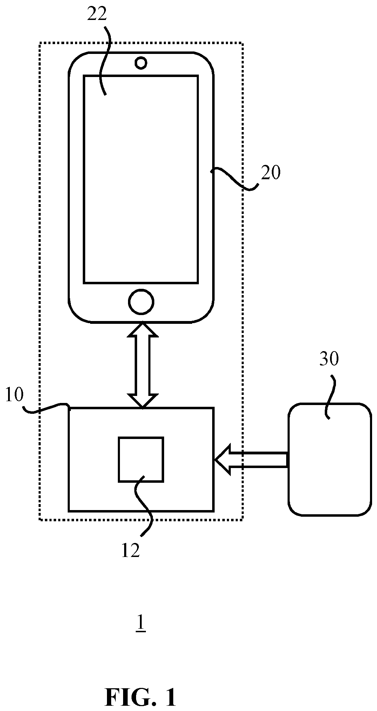

FIG. 1 schematically depicts a sleep monitoring system 1 according to an example embodiment. The sleep monitoring system 1 for example may be configured such that a user of the system can maintain an electronic sleep/wake diary and receive sleep therapy guidance from the system, e.g. CBT-I guidance. To this end, the system 1 may comprise an electronic device 20 comprising a user interface 22 through which the user can maintain this diary and provide information to the sleep monitoring system 1, such as user perceived values of sleep parameters such as sleep onset latency (SOL), wake after sleep onset (WASO), total sleep time (TST) or the like. The user may operate the user interface 22 to provide such information to a processor arrangement 12 of a computer 10, which may update the electronic sleep/wake diary with this information.

In addition, the sleep monitoring system 1 may comprise a sleep monitor 30., The sleep monitor 30 may take any suitable shape or form, such as a wearable sleep monitor or an integrated sleep monitor, e.g. a sleep monitor integrated in a mattress, pillow, bedside cabinet or the like. The sleep monitor 30 provides sleep monitoring data to the processor arrangement 12, which sleep monitoring data is processed by the processor arrangement 12 to extract an objective value of the sleep parameter from the sleep monitoring data. The objective value of the sleep monitoring data is typically extracted from the sleep monitoring data using some recommended data evaluation method, e.g. a recommended threshold value, in order to identify an event during the sleep/wake cycle of the user that corresponds to the sleep parameter. For example, in the case of SOL, the manual of the American Association of Sleep Medicine (AASM) defines SOL as 'the first 15 minutes of consolidated sleep after lights off, and the processor arrangement 12 may be configured to deploy such recommended definitions in order to extract the objective value of the sleep parameter from the sleep monitoring data provided by the sleep monitor 30. Similar definitions have been provided for other sleep parameters such as WASO and TST.

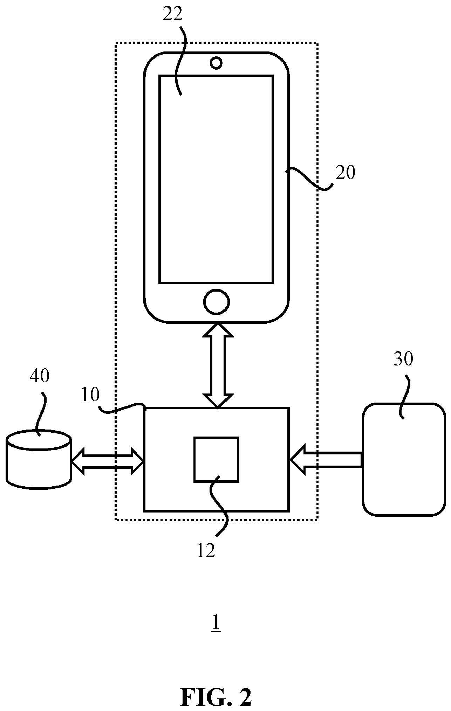

The processor arrangement 12 may update the electronic sleep/wake diary of the user with his or her perceived value of the sleep parameter and the corresponding objective value of the sleep parameter and/or store the received sleep monitoring data in a database 40 as shown in FIG. 2, optionally including the user perceived value and/or the objective value of the sleep parameter. It will be readily understood by the skilled person that the user perceived value and the objective value of the sleep parameter relate to the same sleep event, e.g. to the same point in time during a sleep therapy for instance. The processor arrangement 12 may be adapted to generate an output comprising at least the objective value of the sleep parameter and preferably also the user perceived value of the sleep parameter for this purpose. The output may be stored in the electronic sleep/wake diary and/or may be displayed on the electronic device 20 for information purposes.

The computer system 10 may be a discrete system, e.g. a cloud-based system through which the electronic device 20 and the sleep monitor 30 may connect over a network connection such as the Internet. Alternatively, as indicated by the dashed box, the computer system 10 may form part of the electronic device 20, e.g. the processor arrangement 12 may form part of the electronic device 20. The processor arrangement 12 may contain any suitable number of processors, e.g. one or more processors or processor elements (cores) working together. The electronic device 20 may take any suitable shape or form, e.g. a desktop or laptop computer, or preferably a portable device such as a smartphone, tablet or the like. The user interface 22 may comprise one or more elements, e.g. a touchscreen, a mouse, a keyboard, a display device, a microphone used in conjunction with speech recognition software operating on the electronic device 20 and so on. Any combination of such elements may be contemplated, although in a specific embodiment the electronic device 20 is a portable electronic device and the user interface 22 comprises a touchscreen. The sleep monitor 30 may take any suitable shape or form, e.g. a wrist-worn sleep monitor or a head band, and may produce any suitable form of sleep monitoring data, e.g. one or more of accelerometric data, photoplethysmography (PPG) data, EEG data, polysomnography (PSG) data, and so on. The sleep monitor 30 may communicate with the processor arrangement 12 of the sleep monitoring system 1 in any suitable manner, e.g. using a wireless communication protocol such as Bluetooth or the like, e.g. through a wireless receiver (not shown) of the electronic device 20.

The sleep monitoring system 1 may be used as part of a sleep therapy having a certain duration, e.g. a six-week or eight-week programme, in which the user keeps the sleep/wake diary to keep track of his or her progress. As previously mentioned, the use of a sleep monitor may increase user interaction with the sleep monitoring system, but may not lead to improved therapy outcomes. This is particularly the case for severe insomnia patients. What is more, for paradoxical insomnia patients, the reporting of the objective value of a sleep parameter as extracted from the sleep monitoring data provided by the sleep monitor 30 can lead to non-compliance with the sleep therapy because the discrepancy between the user's own perception of the value of the sleep parameter and the objective value can lead to a loss of trust in the therapy as the user can get the impression that his or her complaints are not recognized by the sleep monitoring system.

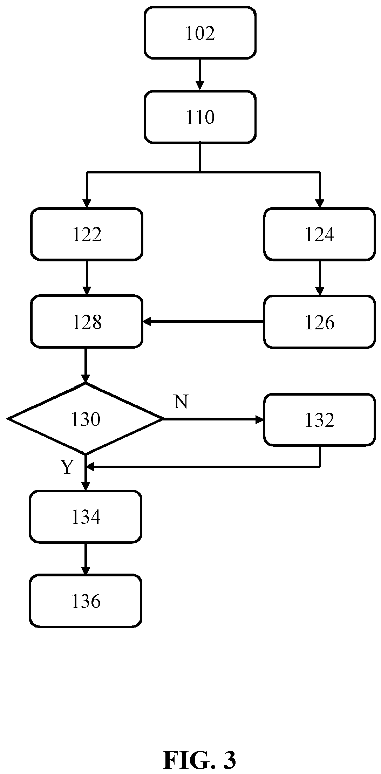

To address this problem, in accordance with certain embodiments of the present invention, a method 100 for implementation by the processor arrangement 12 of the sleep monitoring system 1 is provided, a flowchart of which is shown in FIG. 3. The method 100 starts in operation 102, after which the method 100 may proceed to operation 110, which is a benchmarking or calibration operation that will be explained in further detail below. Alternatively, operation 110 may be skipped and the method 100 may immediately proceed to operation 122 in which the processor arrangement 12 receives a perceived value of a sleep parameter from the user, e.g. through the user interface 22, for a particular sleep event at a point in time during a sleep therapy, whilst in operation 124 the processor arrangement 12 receives the sleep monitoring data from the sleep monitor 30 for the same sleep event, i.e. relating to the same point in time during the sleep therapy. In operation 126, the processor arrangement 12 extracts the objective value of the sleep parameter from the sleep monitoring data received from the sleep monitor 30 and compares the objective value of the sleep parameter to the received user perceived value of the sleep parameter in operation 128.

If it is decided in operation 130 that the comparison result of the objective value of the sleep parameter to the received user perceived value of the sleep parameter shows that the discrepancy between the objective value of the sleep parameter and the received user perceived value of the sleep parameter is small enough such that the user is unlikely to be alarmed by this discrepancy, the method 100 may proceed directly to operation 134 in which the processor arrangement 12 generates an output comprising at least the objective value of the sleep parameter, e.g. for displaying this value to the user on the electronic device 20 and/or for updating the database 40 with the sleep monitoring data and the extracted objected value. This output may further comprise the perceived value of the sleep parameter before the method 100 terminating in operation 136.

On the other hand, if it is decided in operation 130 that the comparison result of the objective value of the sleep parameter to the received user perceived value of the sleep parameter shows that the discrepancy between the objective value of the sleep parameter and the received user perceived value of the sleep parameter is large enough such that the user is likely to be alarmed by this discrepancy and may lose faith in the sleep therapy, the method 100 proceeds to operation 132 in which the objective value of the sleep parameter is augmented into a further value more closely resembling the user-perceived value of the sleep parameter, in which case in operation 134 the further value rather than the objective value is included in the output generated by the processor arrangement 12 presented to the user through the electronic device 20, thereby giving the user the impression that the sleep monitor 30 is accurately reflecting the perceived sleep characteristics of the user.

Preferably, the scale of the augmentation of the objective value of the sleep parameter into the further value is a function of the point in time during the sleep therapy. More specifically, as the sleep therapy progresses, the difference between the further value and the objective value should be reduced such that during the initial stages of the sleep therapy the further value closely resembles the value of the sleep parameter as perceived by the user in order to safeguard user compliance with the sleep therapy, whereas during the latter stages of the sleep therapy, the further value can more closely resemble the objective value, as the therapy is becoming more effective and the user's perception of the value of the sleep parameter more closely resembles the objective or actual value of this parameter.

The augmentation of the objective value of the sleep parameter may be achieved in a number of ways, that is, the objective value of the sleep parameter may be adjusted by a defined amount in order to produce the further value, or the processing of the sleep monitoring data may be altered such that the processing result produces the further value rather than the objective value. Both will be explained in further detail below.

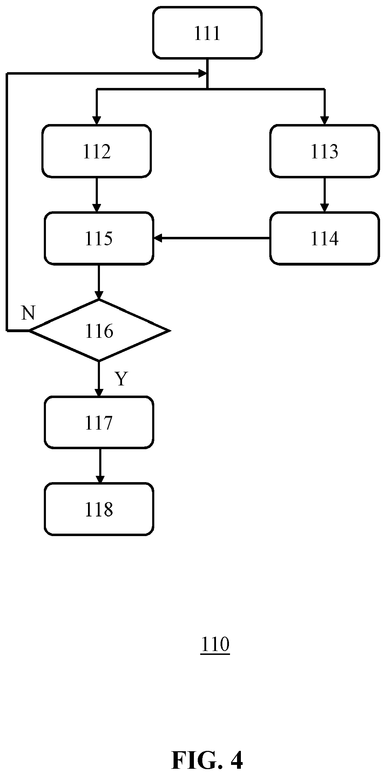

In a first embodiment, an offset to be applied to the objective value of the sleeping parameter in order to yield the augmented value, i.e. the further value, of the sleeping parameter may be obtained during the benchmarking or calibration operation 110, as shown in FIG. 4. During this operation, which starts in operation 111 and may precede the actual sleep therapy, the processor arrangement 12 receives user perceived value of a sleep parameter relating to a particular sleep event in operation 112 and sleep monitoring data pertaining to the same sleep event, i.e. to the same point in time, in operation 113. In operation 114, the processor arrangement 12 extracts the objective value of the sleep parameter from the sleep monitoring data received in operation 113 and in operation 115 stores the received user perceived value of a sleep parameter received in operation 112 and the objective value of the sleep parameter as obtained in operation 114.

Next, it is checked in operation 116 if further data points are to be collected. If this is the case, the calibration or benchmarking process 110 reverts back to operations 112 and 113 respectively to obtain another user perceived value of the sleep parameter and another instance of the sleep monitoring data from the sleep monitor 30 for a different point in time, i.e. a different sleep event. This data acquisition is repeated until a target number of data points has been acquired, e.g. over a defined period of time, after which the calibration or benchmarking process 110 proceeds to operation 117 in which the processor arrangement 12 determines a difference between the acquired user perceived values and the objective values of the sleep parameter for each of said points in time, i.e. sleep events, during the calibration or benchmarking operation prior to the calibration or benchmarking operation terminating in operation 118. The thus established difference is then used during the sleep therapy to augment the objective value of the sleep parameter into the further value as explained above. The determined difference for instance may be an average difference, a median difference or a maximum difference between the user perceived values and the objective values of the sleep parameter as acquired during the calibration or benchmarking operation 110.

In an alternative embodiment, the objective value of the sleep parameter may be augmented during the sleep therapy based on available therapy results in the database 40. The database 40 may store therapy outcomes of a range of severities of paradoxical insomnia, in which for each of these ranges different adjustments of the objective value of the sleeping parameter as derived from the sleep monitoring data provided by the sleep monitor 30 are stored, thereby providing a direct correlation between the severity of the paradoxical insomnia, the applied augmentation of the objective value of the sleep parameter and the therapy outcome, such that upon accessing the database 40 the processor arrangement 12 may select the most appropriate value for the augmentation of the objective value of the sleep parameter obtained in operation 126 of the method 100 based on the difference between the user perceived value and the objective value of this parameter as obtained in operation 128 of the method 100. In a further refinement, the selection of the appropriate adjustment of the objective value of the sleep parameter into the further value of the sleep parameter from the database 40 may further take patient characteristics matching the type of sleep disorder of the user into consideration, such as phenotypes or subtypes of insomniacs. Such information for example may be extracted from the database 40 using readily available artificial intelligence techniques.

In yet another embodiment, the knowledge that characteristics of the sleep monitoring data acquisition by certain sleep monitors 30 lead to a systematic error in the objective value of the sleep parameter extracted from this sleep monitoring data, i.e. a systematic discrepancy between the objective value of the sleep parameter and its actual value, may be leveraged to augment the objective value with the magnitude of this systematic error to reduce the discrepancy between the user perceived value and the objective value of the sleep parameter if it is known which sleep monitor 30 is attached to the sleep monitoring system 1. Of course, the objective value of the sleep parameter may be further augmented using any of the aforementioned augmentation principles to further reduce this discrepancy.

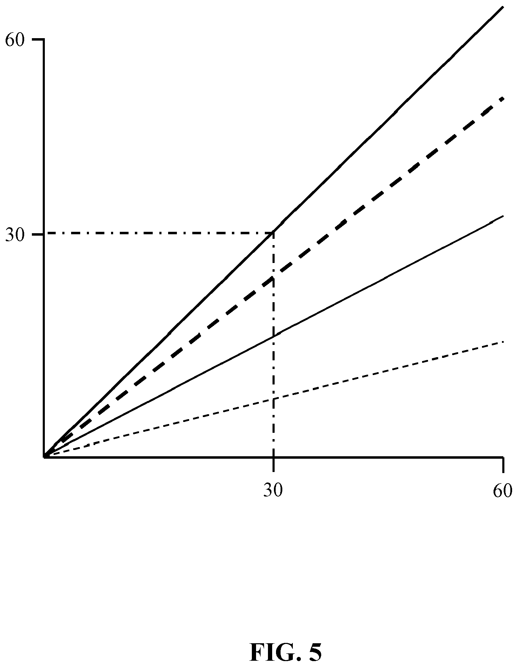

FIG. 5 schematically depicts a graph in which a simulated measured SOL in minutes is shown on the y-axis and the perceived SOL in minutes in shown on the x-axis, with the solid lines depicting the measured results with an EEG-based sleep monitor for a healthy sleeper (thick line) and a paradoxical insomniac (thin line) and the dashed lines depicting the measured results with a wearable accelerometry-based sleep monitor for a healthy sleeper (thick line) and a paradoxical insomniac (thin line), which clearly demonstrates that for paradoxical insomniacs, the amount of correction that needs to applied to the value of the sleep parameter derived from sleep monitoring data depends on the type of sleep monitoring device used.

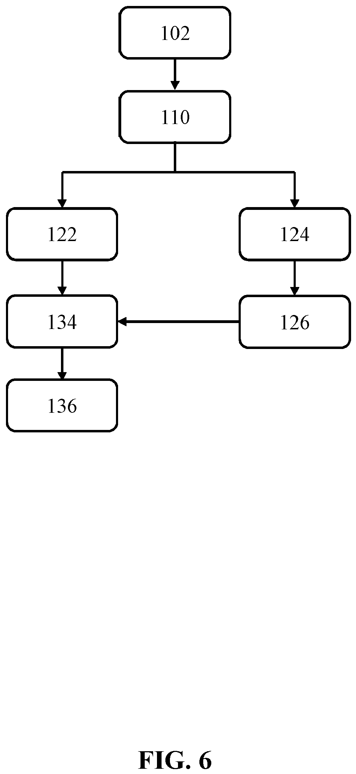

In another embodiment, rather than applying a correction to the objective value of the sleep parameter derived from the sleep monitoring data provided by the sleep monitor 30, the way the sleep monitoring data is processed in operation 126 may be altered to obtain the further data value rather than the objective data value of the sleep parameter in order to reduce the discrepancy between the user perceived value of the sleep parameter and the value of this parameter obtained with the sleep monitor. This is explained in more detail with the aid of FIG. 6, which depicts a flowchart of the method 100 in accordance with this embodiment. As before, the method starts in operation 102 and may proceed to operation 110 in which the difference between the objective value of the sleep parameter as obtained with the sleep monitor 30 and the user perceived value of the sleep monitor for the same sleep event may be quantified in a calibration or benchmark process, as previously explained. The method 100 then proceeds to previously described operations 122 and 124 in which the user perceived value of the sleep parameter for a sleep event at a particular point in time, e.g. during the sleep therapy, and the sleep monitoring data for the same sleep event are respectively received by the processor arrangement 12.

In operation 126, rather than evaluating the sleep monitoring data using a recommended criterion or threshold value, e.g. 15 minutes consolidated sleep from lights off to determine the value of SOL as recommended by the AASM, the evaluation criterion, e.g. threshold value, is adjusted such that the value of the sleep parameter extracted from the sleep monitoring data by the processor arrangement 12 more closely resembles the user perceived value of this parameter, at least during the initial stages of the sleep therapy, e.g. by increasing the number of minutes of consolidated sleep from lights off to determine the value of SOL. As before, this evaluation criterion is gradually adapted over the course of the sleep therapy such that towards the latter stages of the sleep therapy the applied evaluation criterion, e.g. threshold value, closely resembles or matches the recommended value of this criterion. For example, for a six-week sleep therapy programme, the AASM guidelines may be adjusted as shown in Table 1 below.

**Table 1**

| | Week 1 | Week 2 | Week 3 | Week 4 | Week 5 | Week 6 |
|---|---|---|---|---|---|---|
| AASM | 15 | 15 | 15 | 15 | 15 | 15 |
| Guidelines | | | | | | |
| Adjusted Guidelines | 30 | 25 | 20 | 15 | 15 | 15 |

The appropriate level of adjustment of the recommended criterion for the determination of the sleep parameter may be determined in any suitable manner. For example, the established difference between the user perceived value and the objective value of the sleep parameter during the calibration or benchmarking operation 110 may be used to determine the personally adjusted criterion for the evaluation of the sleep monitoring data in order to produce an augmented objective value, i.e. the further value, of the sleep parameter that more closely resembles the user perceived value of the sleep parameter. Upon generation of the further value of the sleep parameter in operation 126, the method 100 proceeds to previously described operation 134, after which the method 100 terminates in operation 136.

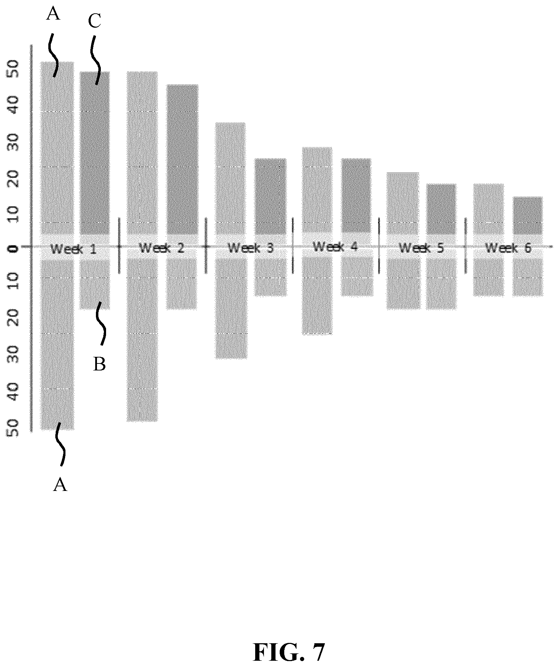

The principle of at least some of the embodiments of the present invention are graphically depicted in FIG. 7-9. FIG. 7 shows the user perceived values of SOL (labelled A), the objective values of SOL (labelled B) and the further values of SOL (labelled C) obtained by augmenting the objective values of SOL in accordance with the teachings of the present invention (y-axis) over the course of a 6-week sleep therapy programme (x-axis). Below the x-axis the comparison between the user perceived values of SOL (labelled A) and the objective values of SOL (labelled B) during this therapy is shown, whereas above the x-axis the comparison between the user perceived values of SOL (labelled A) and the further values of SOL (labelled C) during this therapy is shown.

As can be seen, during the first three weeks of the therapy programme, a large discrepancy between the user perceived values of SOL and the objective values of SOL exists, which discrepancy is largely removed when comparing the user perceived values of SOL and the further values of SOL due to the augmentation of the objective values of SOL into these further values in order to increase user confidence in the therapy programme. However, as the therapy is becoming more effective, the agreement between the user perceived values of SOL and the objective values of SOL dramatically increases (week 5 and week 6) such that a much smaller augmentation of the objective values of SOL into the further values of SOL can be applied without eroding user confidence. In some embodiments, no augmentation of the objective value of the sleep parameter is required during the latter stages of the sleep therapy programme for this reason, as for instance shown in Table 1.

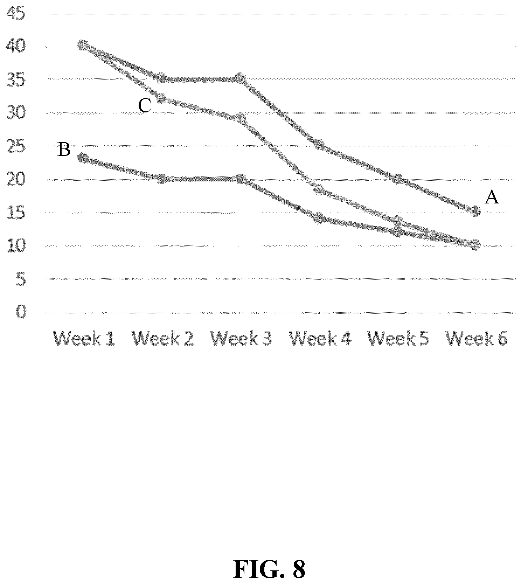

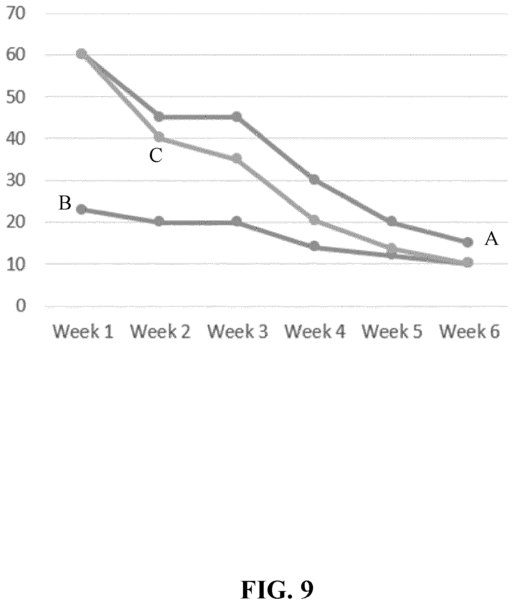

FIG. 8 depicts a graph showing evolution of SOL for a patient having an initial disagreement between user-perceived SOL (labelled A) and objective SOL (labelled B) of 17 minutes and FIG. 9 depicts a graph showing evolution of SOL for a patient having an initial disagreement between user-perceived SOL (labelled A) and objective SOL (labelled B) of 37 minutes during a 6-week sleep therapy programme. The augmented objective SOL, i.e., the further value of SOL is labelled C in these graphs. Both graphs demonstrate that the approach according to at least some embodiments of the present invention ensures that the discrepancy between the user-perceived SOL and the objective SOL is reduced during the course of the therapy, reaching good agreement between the two at the end of the sleep therapy programme.

The above described embodiments of the method 100 executed by the processor arrangement 12 may be realized by computer readable program instructions embodied on a computer readable storage medium having, when executed on a processor arrangement 12 of a computing device 10, e.g. the electronic device 20 comprising the processor arrangement 12, cause the processor arrangement 12 to implement any embodiment of the method 100. Any suitable computer readable storage medium may be used for this purpose, such as for example an optically readable medium such as a CD, DVD or Blu-Ray disc, a magnetically readable medium such as a hard disk, an electronic data storage device such as a memory stick or the like, and so on. The computer readable storage medium may be a medium that is accessible over a network such as the Internet, such that the computer readable program instructions may be accessed over the network. For example, the computer readable storage medium may be a network-attached storage device, a storage area network, cloud storage or the like. The computer readable storage medium may be an Internet-accessible service from which the computer readable program instructions may be obtained. In an embodiment, the portable computing device 50 is adapted to retrieve the computer readable program instructions from such a computer readable storage medium and to create a new computer readable storage medium by storing the retrieved computer readable program instructions in a data storage arrangement of the computing device 10, e.g. in a memory device or the like forming part of the portable computing device 10.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A computer-implemented method (100) of producing a sleep evaluation for a user for use with a sleep therapy, the method comprising, with a processor arrangement (12):
receiving (122) a perceived value of a sleep parameter from the user, said sleep parameter corresponding to sleep behavior of said user at a point in time during said sleep therapy;
receiving (124) sleep monitoring data from an electronic sleep monitor, said sleep monitoring data incorporating an objective value of said sleep parameter at said point in time; and, in case of the perceived value being different to the objective value:
processing the sleep monitoring data such as to augment (132) said objective value into a further value of said sleep parameter to reduce the discrepancy between the perceived value and the objective value; and
producing (134) a sleep evaluation output for the user comprising at least the further value of said sleep parameter.

2. The computer-implemented method (100) of claim 1, wherein the sleep evaluation output for the user comprises the perceived value of the sleep parameter and the augmented further value of the sleep parameter.

3. The computer-implemented method (100) of claim 1 or 2, wherein said augmenting is a function of the point in time during said sleep therapy such that an amount by which said objective value is augmented into the further value is reduced over time.

4. The computer-implemented method (100) of any of claims 1-3, wherein augmenting (132) the objective value of said sleep parameter into the further value comprises augmenting a threshold value for recognizing a data pattern in the sleep monitoring data from which the objective value of the sleep parameter is determined.

5. The computer-implemented method (100) of claim 4, wherein augmenting (132) said threshold value comprises altering a recommended threshold value into a further threshold value producing a further value of the sleep parameter that more closely resembles the perceived value of the sleep parameter compared to the objective value of the sleep parameter produced with the recommended threshold value.

6. The computer-implemented method (100) of any of claims 1-5, further comprising:
receiving (112) a series of further perceived values of a sleep parameter from the user, each of said further perceived values of said sleep parameter corresponding to sleep behavior of said user at a different point in time during a calibration period preceding said sleep therapy;
receiving (113) a series of further sleep monitoring data from the electronic sleep monitor, each instance of said further sleep monitoring data corresponding to sleep behavior of said user at one of said different points in time during said calibration period;
extracting (114) a further objective value of said sleep parameter from each instance of the received sleep monitoring data;
determining (115) a difference between the further perceived values and the further objective values of the sleep parameter for each of said points in time during said calibration period; and
augmenting (132) the objective value of said sleep parameter based on said determined difference.

7. The computer-implemented method (100) of claim 6, wherein the determined difference is one of an average difference, a maximum difference and a median difference.

8. The computer-implemented method (100) of any of claims 1-5, wherein augmenting (132) the objective value of said sleep parameter into the further value comprises retrieving an optimal further value from a database containing sleep therapy outcomes as a function of different further values used for an actual difference between the perceived value and the objective value of the sleep parameter in said sleep therapies.

9. The computer-implemented method (100) of claim 8, further comprising retrieving the optimal further value from a database containing sleep therapy outcomes as a function of different further values used for:
the actual difference between the perceived value and the objective value of the sleep parameter in different sleep therapies; and
patient characteristics matching the type of sleep disorder of the user.

10. The computer-implemented method (100) of any of claims 1-9, wherein augmenting (132) the objective value of said sleep parameter into the further value is at least in part based on a known systematic inaccuracy in the sleep monitoring data produced by the sleep monitor.

11. The computer-implemented method (100) of any of claims 1-10, wherein the sleep parameter is one of sleep onset latency, total sleep time and wake after sleep onset.

12. A computer program product comprising a computer readable storage medium having computer readable program instructions embodied therewith for, when executed on the processor arrangement (12), cause the processor arrangement to implement the method (100) of any of claims 1-11.

13. A sleep monitoring system (1) comprising a processor arrangement (12) and the computer program product of claim 12, wherein the processor arrangement is adapted to execute the computer readable program instructions of said computer program product.

14. The sleep monitoring system (1) of claim 13, further comprising at least one of:
a user interface (22) adapted to be communicatively coupled to the processor arrangement (12) for providing the processor arrangement with a perceived value of a sleep parameter from a user, said sleep parameter corresponding to sleep behavior of said user at a point in time during a sleep therapy; and
a sleep monitor (30) adapted to be communicatively coupled to the processor arrangement for providing the processor arrangement with sleep monitoring data corresponding to sleep behavior of said user at said point in time.

15. The sleep monitoring system (1) of claim 14, wherein the processor arrangement (12) and the user interface (22) form part of the same electronic device (20).
